(19) Europäisches Patentamt
European Patent Office
Office européen des brevets

(11) EP 4 455 278 A1

(12) **EUROPEAN PATENT APPLICATION**
published in accordance with Art. 153(4) EPC

(43) Date of publication:
**30.10.2024 Bulletin 2024/44**

(21) Application number: **22912060.5**

(22) Date of filing: **26.12.2022**

(51) International Patent Classification (IPC):
***C12N 9/10*** *(2006.01)* ***C12P 19/56*** *(2006.01)*
***C12P 19/18*** *(2006.01)* ***C12N 15/62*** *(2006.01)*
***C12N 15/81*** *(2006.01)*

(52) Cooperative Patent Classification (CPC):
**C12N 9/10; C12N 15/62; C12N 15/81; C12P 19/18; C12P 19/56**

(86) International application number:
**PCT/KR2022/021327**

(87) International publication number:
**WO 2023/121427 (29.06.2023 Gazette 2023/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **24.12.2021 KR 20210187435**

(71) Applicant: **Samyang Corporation**
**Jongno-gu**
**Seoul 03129 (KR)**

(72) Inventors:
- **KIM, Jeongmin**
  **Yongin-si, Gyeonggi-do 16826 (KR)**
- **KO, Hyeokjin**
  **Suwon-si, Gyeonggi-do 16488 (KR)**
- **MOON, Junho**
  **Seongnam-si, Gyeonggi-do 13588 (KR)**
- **CHOI, Eunsoo**
  **Seongnam-si, Gyeonggi-do 13588 (KR)**

(74) Representative: **Schön, Christoph**
**Dr. Schön, Neymeyr & Partner mbB**
**Bavariaring 26**
**80336 München (DE)**

Remarks:
The complete document including Reference Table(s) and the Sequence Listing(s) can be downloaded from the EPO website

(54) **GLYCOSYLTRANSFERASE VARIANT, AND METHOD OF PREPARING STEVIOL GLYCOSIDES USING SAME**

(57) The present invention relates to a glycosyltransferase mutant, and a composition and a method for producing steviol glycosides using the same, and more specifically, to a glycosyltransferase of rebaudioside that transfers glucose to steviol glycosides and its mutant, a recombinant strain expressing the enzymes, and a method for producing rebaudioside using the same.

EP 4 455 278 A1

【FIG. 2】

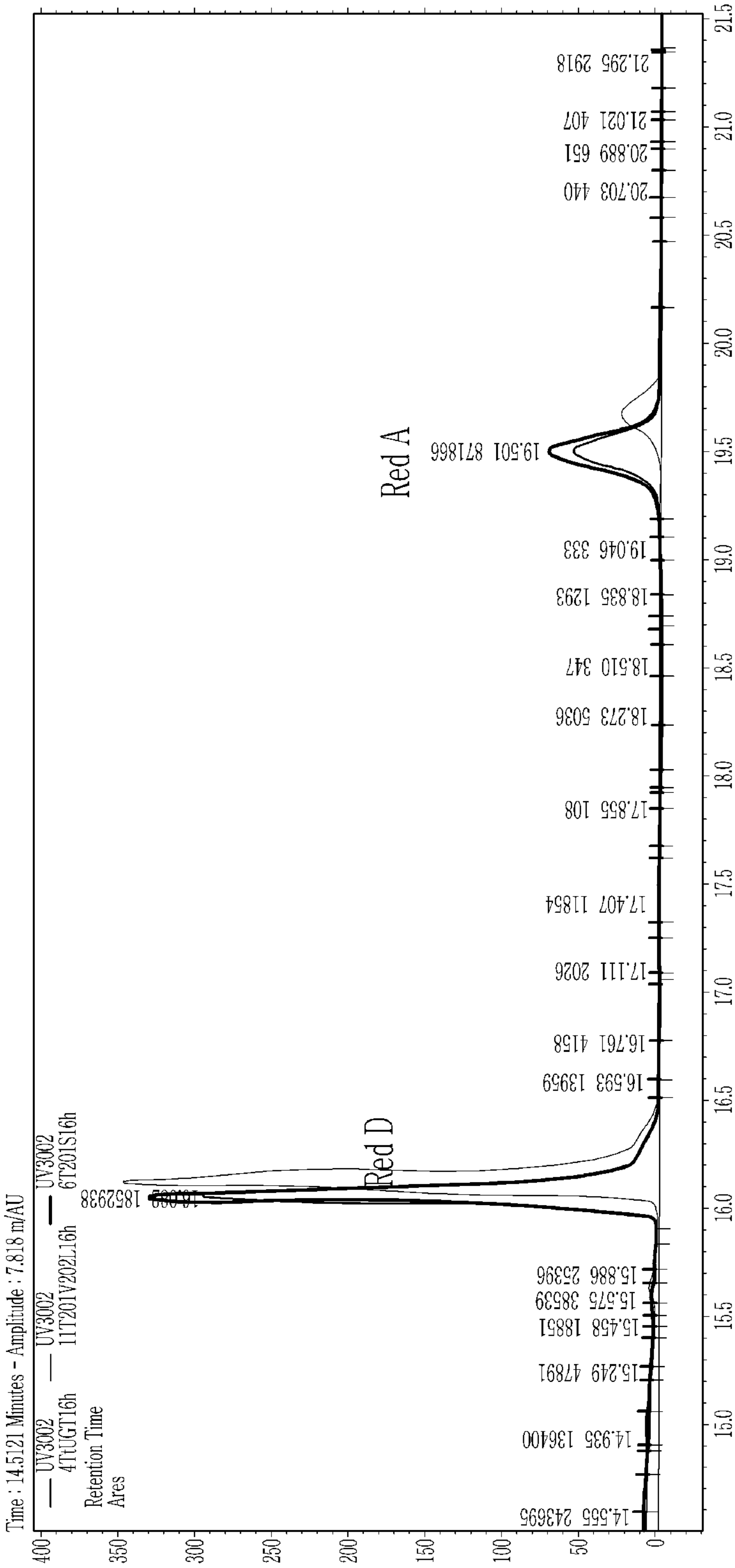
Time : 14.5121 Minutes - Amplitude : 7.818 m/AU
UV3002 4TrUGT16h
UV3002 11T201V202L16h
UV3002 6T201S16h
Retention Time
Ares
Red D
Red A
14.555 243695
14.935 136400
15.249 47891
15.458 18851
15.575 38539
15.886 25396
16.593 13959
16.761 4158
17.111 2026
17.407 11854
17.855 108
18.273 5036
18.510 347
18.835 1293
19.046 333
19.501 871866
20.703 440
20.889 651
21.021 407
21.295 2918

## Description

[TECHNICAL FIELD]

The present invention relates to a glycosyltransferase, and a composition and a method for producing steviol glycosides using the same, and more specifically, to a glycosyltransferase that transfers glucose to steviol glycosides, recombinant strains expressing the enzyme, and methods for producing rebaudioside using the same.

[RELATED ART]

Sweeteners are some of the most common ingredients in the food, beverage, or confectionery industry. Sweeteners can be incorporated into the final food product during production or, when properly diluted for stand-alone use, can be used as table sweeteners or as a household substitute for sugar in baking. Sweeteners include natural sweeteners, such as, for example, sucrose, high fructose corn syrup, molasses, maple syrup, and honey, and artificial sweeteners, such as, for example, aspartame, saccharin, and sucralose.
Stevia extract is a natural sweetener that can be extracted from the perennial shrub, Stevia rebaudiana. Stevia extract, purified to various levels, is used as highly intense seasoning in food and mixture or as table-top sweetener.
Extracts of the stevia plants contain rebaudiosides and other steviol glycosides which contribute to their sweet taste, but conventional commercial products are mainly Rebaudioside A, with small amounts of other glycosides such as Rebaudioside C, D, and F. Plant-derived stevia extracts can contain contaminants such as derived compounds that cause off-flavors. These off-flavors can be problematic depending on the food system or application chosen.
In addition, the composition of stevia extracts can vary widely depending on the soil and climate in which the plant is grown. Depending on the source plant, climatic conditions, and extraction process, the amount of rebaudioside A in commercial preparation is reported to vary from 20 to 97% of the total steviol glycoside content. Other steviol glycosides are also present in varying amounts in stevia extracts.
Stevia extract produced from the stevia plant contains several steviol glycosides and compounds that cause off-flavor compounds, and their elimination or purification is labor intensive and inefficient. Accordingly, there is a need for a recombinant production system that can produce and accumulate steviol glycosides such as Reb D and Reb M at high yields.
In particular, Reb M is 200 to 350 times sweeter than sugar and has a clean sweet taste with a slightly bitter or licorice-like aftertaste. Recently, the EFSA Panel on Food Additives and Flavorings announced that Reb M is safe as a food additive. Because Reb M is suitable for blending and applicable to various food and beverage products, Reb M may attract great interest and generate continued demand as a natural sweetener in the global food industry. For this purpose, the development of highly efficient enzymes is still required. In addition, there is still a need for improved production of Reb D for production of the steviol glycoside, Reb M, in recombinant hosts for commercial use.

[DISCLOSURE]

[TECHNICAL PROBLEM]

An embodiment of the present invention relates to a glycosyltransferase enzyme transferring glucose to steviol glycosides, a nucleotide molecule encoding the enzyme protein, and a recombinant vector and a transformed microorganism including the nucleotide molecule.
An embodiment of the present invention relates to a composition for producing steviol glycosides, including at least one selected from the group consisting of a glycosyltransferase enzyme transferring glucose to steviol glycosides, a recombinant microorganism expressing the enzyme protein, microbial cells of the microorganism, cultures of the microorganism, lysates of the microorganism, and extracts thereof.
An embodiment of the present invention is to provide a method of producing steviol glycosides, including a step of reacting at least one selected from the group consisting of a glycosyltransferase enzyme transferring glucose to steviol glycosides, a recombinant microorganism expressing the enzyme protein, microbial cells of the microorganism, cultures of the microorganism, lysates of the microorganism, and extracts thereof, with a substrate of the enzyme.
An embodiment of the present invention is to provide a method of producing steviol glycosides through transferring glucose with supplying UDP_Glucose, which is an active form of glucose as a cofactor required for transferring glucose to steviol glycoside.
A further embodiment of the present invention is to provide a method of producing steviol glycosides through transferring glucose, by supplying UDP and sucrose and further including sucrose synthase in order to supply an active form of glucose as a cofactor required for transferring glucose to steviol glycoside.

[TECHNICAL SOLUTION]

Hereinafter, the present invention will be described in more detail.

An example of the present invention relates to a glycosyltransferase that transfers glucose to steviol glycosides, and more specifically, UDP-glucosyltransferase (i.e., uridine diphosphate glucosyltransferase, abbreviated as UGT).

Particularity, the UDP-glucoslyltransferase of the present invention can be mutant enzyme in which at least one amino acid selected from the group consisting of the $201^{st}$ and $202^{nd}$ amino acids from the N-terminus in an amino acid sequence of SEQ ID NO: 1 is substituted with at least one amino acid selected from the group consisting of serine and valine. More specifically, the mutant enzyme can include a substitution of serine for threonine at the $201^{st}$ position from the N-terminus in an amino acid sequence of SEQ ID NO: 1, a substitution of leucine for valine at the $202^{nd}$ position from the N-terminus in an amino acid sequence of SEQ ID NO: 1, or a substitution of serine for threonine at the $201^{st}$ position and a substitution of leucine for valine at the $202^{nd}$ position, and more specifically an amino acid sequence of SEQ ID NO: 3.

Additionally, the polynucleotide sequence of mutant enzyme can be a mutant enzyme including a polynucleotide sequence encoding an amino acid sequence which contains a substitution of serine for threonine at the $201^{st}$ position from the N-terminus in an amino acid sequence of SEQ ID NO: 1, a substitution of leucine for valine at the $202^{nd}$ position from the N-terminus in an amino acid sequence of SEQ ID NO: 1, or a substitution of serine for threonine at the $201^{st}$ position and a substitution of leucine for valine at the $202^{nd}$ position, and more specifically an amino acid sequence of SEQ ID NO: 3. The mutant enzyme includes an amino acid sequence of SEQ ID NO: 3 or a polynucleotide sequence encoding the amino acid sequence.

The mutant enzyme of TaUGTm (T201S) in the present invention includes a substitution of Ser for Thr at position 201 from the N-terminus in an amino acid sequence of SEQ ID NO: 1 for the wild-type enzyme, for example, a substitution of TCA for ACC, TaUGTm (V202L) includes a substitution of Leu for Val at position 202, for example, a substitution of TTA for GTA, or TaUGTm (T201 SV202L) is includes a substitution of Ser-Leu for Thr-Val at positions 201 and 202 from the N-terminus in an amino acid sequence of SEQ ID NO: 1 for the wild-type enzyme, and more specifically, a substitution of TCATTA for ACCGTA in a polynucleotide sequence of SEQ ID NO: 2.

According to another example of the present invention, the mutant UDP-glucosyltransferase may include an amino acid sequence having a sequence identity of 92% or more, 95% or more, 97% or more, or 99% or more. As long as it is an amino acid sequence showing activity corresponding to that of the enzyme protein, it also includes variant enzyme protein having deleted, modified, substituted, or added part of the amino acid sequence. However, the mutant UDP-glucosyltransferase does not include the wild-type enzyme consisting of an amino acid sequence of SEQ ID NO: 1.

The variant enzyme has one encoded by a polynucleotide sequence having a sequence identity of 40% or more, 50% or more, 60% or more, 70% or more, 80% or more, 90% or more, 95% or more, 97% or more, or 99% or more with the polynucleotide sequence encoding the variant UDP-glucosyltransferase. % or more, 97% or more, or 99% or more may be encoded by a polynucleotide sequence. However, the mutant UDP-glucosyltransferase does not include the wild-type enzyme consisting of the polynucleotide sequence of SEQ ID NO: 2.

In the above, the term "homology" or "identity" refers to the degree of matching with a given amino acid sequence or nucleotide sequence and can be expressed as a percentage. Herein, homologous sequence having the same or similar activity corresponding to a given amino acid sequence or nucleotide sequence are expressed as "% homology" or "% identity."

The variant enzyme has an activity of 101% or higher, for example, 101% to 200%, based on 100% of the activity of converting the Rebaudioside A (Reb A) substrate to Rebaudioside D (Reb D) of the wild-type enzyme containing an amino acid sequence of SEQ ID NO: 1. Namely, the production rate of Reb D and Reb E can be improved by modifying the enzyme through mutation in order to improve the activity of the enzyme, and can be reacted with a second UDP-glucosyltransferase, for example, UGT76G1 derived from stevia, to produce Rebaudioside M (Reb M). Accordingly, the productivity of Reb D and Reb E can be increased through an enzymatic conversion reaction, and using this, a highly functional sweetener called Reb M can be produced with high productivity.

The UDP-glucosyltransferase according to the present invention has an activity converting at least one substrate selected from the group consisting of stevioside and Reb A into steviol glycosides, such as at least one selected from the group consisting of Reb D and Reb E in the presence of a glucose donor. More specifically, it can convert Reb A into Reb D and stevioside into Reb E (see Scheme 1 below), and has conversion activity for single substrate or mixed substrates of Reb A and stevioside.

[Scheme 1]

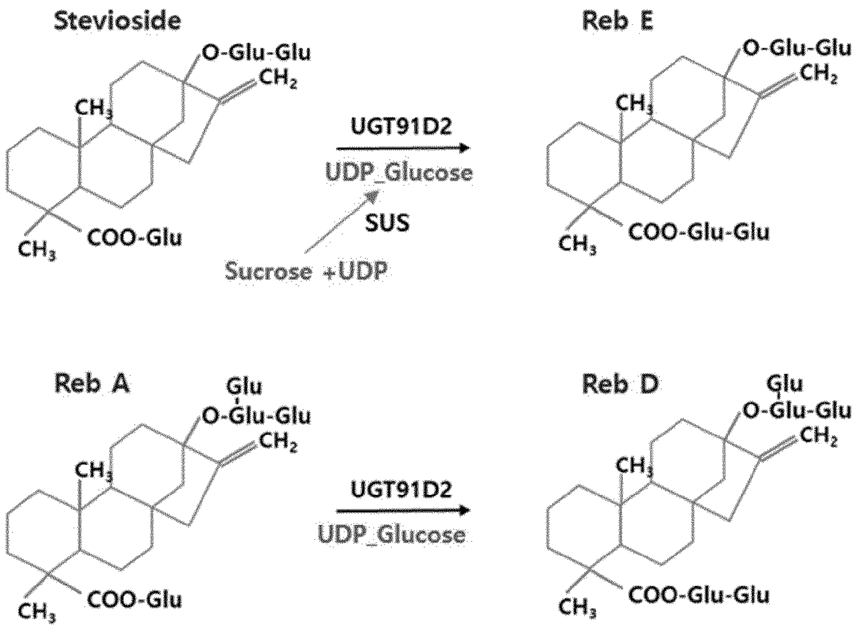


As shown in Scheme 1, the conversion from Reb A to Reb D involves transferring glucose to the steviol ring through the beta-1,2 glyosidic linkage, so that it can mainly produce the steviol glycosides glycosylated at position 19 of the steviol ring. Steviol glycosides may include one to three glucose molecules bonded through β-linkage. Steviol glycosides are compounds found in the leaves of *Stevia rebaudiana Bertoni*, which are structurally characterized by the single base steviol, which differs by the presence of saccharide residues at the positions of C13 and C19. Steviol glycosides generally include, but are not limited to, stevioside, rebaudioside A (Reb A), rebaudioside B, C, D, E, F, and M.

The conversion reaction from Reb A to Reb D occurs by adding a glucose molecule by UGT enzyme. The glucose required for this reaction is UDP-glucose, which is an active form, and UGT enzyme transfers glucose from UDP-glucose to produce steviol glycoside. Therefore, the present invention provides a fusion protein of UDP-glucosyltransferase and Sucrose Synthase (SUS) enzyme capable of producing UDP-glucose.

Preferably, UDP-glucose is an expensive co-substrate and needs to be supplied more inexpensively. Sucrose Synthase (SUS) is an enzyme that decomposes sucrose to produce fructose and UDP-glucose. Therefore, by adding SUS enzyme to the reaction of converting Reb A to Reb D or converting stevioside to Reb E, supplement of UDP and sucrose finally provides UGT enzyme with UDP-Glucose, resulting in performing the reaction with more economically producing UDP-Glucose. The SUS enzyme may be provided as such in a composition for producing steviol glycosides containing UDP-glucosyltransferase of the present invention, or in the form of a fusion protein with UDP-glucosyltransferase of the present invention.

For example, if the conversion reaction is performed with a fusion protein of UGT and SUS, two reactions can form a single channel so that enzymatic reactions can occur in batches, resulting in an effective conversion reaction. In addition, when using the TaUGT_SUS fusion enzyme, it is possible to perform the conversion using 1/10 of UDP amount for substrate, and by using UDP at an amount lower than 1/100 of UDP amount for RA40 substrate. Through the conversion reaction using the fusion enzyme, the substrate conversion can be performed economically and high-concentration substrate reaction can be carried out to produce steviol glycosides.

The sucrose synthase may be a sucrose synthase derived from one or more microorganisms selected from the group consisting of *Arabidopsis thaliana, Solanum lycopersicum, Glycine max, Nicotiana tabacum,* and *Thermosynechococcus elongates,* but is not limited thereto. More specifically, the sucrose synthase may be the SUS1 enzyme derived from *Arabidopsis thaliana,* and may include, for example, an amino acid sequence of SEQ ID NO: 4, and a polynucleotide sequence encoding the amino acid sequence of SEQ ID NO: 4, or a polynucleotide sequence of SEQ ID NO: 5.

Sucrose synthase may be fused directly or through a linker to UDP-glucosyltransferase according to the present invention. The linker may be a peptide consisting of 4 to 15 amino acids, or specifically, 4 to 15 amino acids of at least an amino acid selected from the group consisting of G (Gly), S (Ser) and P (Pro), and more specifically may be GGGS (SEQ ID NO: 7), GGGGS (SEQ ID NO: 8), GGGSGGGGS (SEQ ID NO: 9), GGGGSGGGGSGGGGS (SEQ ID NO: 10),

or GGGGPSPGGGGS (SEQ ID NO: 11).

In a specific example of the present invention, it may be a fusion protein that the UDP-glucosyltransferase of the present invention can be linked to the SUS1 enzyme derived from *Arabidopsis thaliana* through a linker (GGGGSG), and specifically includes an amino acid sequence of SEQ ID NO: 7, or a polynucleotide sequence encoding an amino acid sequence of SEQ ID NO: 7 or a polynucleotide sequence of SEQ ID NO: 8.

The variant UDP-glucosyltransferase according to the present invention has an conversion activity of 45% by weight or more of Reb A into Reb D in an enzymatic reaction for 2 to 24 hours, or specifically an conversion activity of 45% by weight or more, 50% by weight or more, 70% by weight or more, 75% by weight or more, 80% by weight or more, 85% by weight or more, or 95% by weight or more of Reb A to Reb D, or more preferably, an conversion activity of 70 % by weight or more, 75% by weight or more, 80% by weight or more, 85% by weight or more, or 95% by weight or more of Reb A to Reb D. More specifically, the conversion activity of the enzyme may be measured under conditions of 30°C, pH 7.2, and 150 rpm. The Reb D conversion rate (%) is calculated by Equation 1 below, and more details are described in Example 4.

【Equation 1】

Reb D Conversion rate (%) = HPLC peak area of Reb D /total HPLC peak area

The variant UDP-glucosyltransferase of the present invention, compared to the wild-type enzyme having an amino acid sequence of SEQ ID NO: 1, has 101% or more, 103% or more, 105% or more, 107% or more, 108% or more, 109% or more, or 110% or more, or preferably 105% or more, 107% or more, 108% or more, or 109% or more, 110% or more, 115% or more, 120% or more, or 125% or more of a relative conversion rate of Reb D, which is an activity of converting Reb A to Reb D in an enzymatic reaction for 2 to 24 hours. The conversion activity of the enzyme may be measured at 30°C, pH 7.2, and 150 rpm. The Reb D conversion rate of the enzyme, which converts to Red D from Reb A as single substrate, is calculated according to Equation 1 below. Specific measurement conditions are described in Example 4 below.

In addition, the modified UDP-glucosyltransferase has an activity (sum of Reb D/E conversion rates) of converting 50 % by weight or more, 60 % by weight or more, 70 % by weight or more, 79 % by weight or more, 80 % by weight or more, 81 % by weight or more, 82 % by weight or more, 83 % by weight or more, 85 % by weight or more, or 87 % by weight or more of the mixed substrate including stevioside and RebA, to Reb D and Reb E(Reb D/E total conversion) in an enzymatic reaction for 2 to 24 hours. More specifically, the conversion activity of the enzyme may be measured under conditions of 30°C, pH 7.2, and 150 rpm.

The modified UDP-glucosyltransferase according to the present invention, compared to the wild-type enzyme having an amino acid sequence of SEQ ID NO: 1, has 103% or more, 105% or more, 106% or more, 108% or more, 109% or more, or 110% or more, or more preferably 105% or more, 107% or more, 108% or more, or 109% or more, 110% or more of total Reb D/E relative conversion rate that the total Reb D/E conversion rate is as a sum of activities for converting Reb A to Reb D and converting stevioside to Reb E, an activity converting 50 % by weight of mixed substrates containing stevioside and Reb A into Reb D and Reb E in an enzymatic reaction for 2 to 24 hours in the mixed substrates containing stevioside and Reb A. More specifically, the conversion activity of the enzyme may be measured using the mixed substrates containing rebaudioside A: STE (stevioside) = 40:60 under conditions of 30°C, pH 7.2, and 150 rpm. The total Reb D/E relative conversion rate of the enzyme refers to the total Reb D/E conversion rate of the enzyme, based on 100% of the total Reb D/E conversion rate of the wild-type enzyme. In other words, the total Reb D/E conversion rate of the enzyme for converting the mixed substrate to Reb D/E is calculated according to Formula 2 below. The relative total Reb D/E conversion rate of the enzyme refers to the total Reb D/E conversion rate of the mutant enzyme of TaUGT calculated based on 100% of total Reb D/E conversion rate of the wild type enzyme.

Reb D/E conversion rate (%) = (HPLC peak area of Reb D/E)/(total HPLC peak area) [Equation 2]

The wild-type enzyme of UDP-glucosyltransferase according to the present invention may be an enzyme derived from *Triticum aestivum,* which is obtained by searching for plant-driven enzymes. By identifying the protein function, the enzyme has UGT enzyme activity, and the enzyme activity of converting stevioside or Reb A into Reb E or Reb D, respectively.

Reb D with high sweetness is a saccharide in small amounts in the stevia plant and is produced from Reb A, in which the known enzyme involved in this production process is UGT91D2 of the stevia plant. An enzyme with similar activity is EUGT11 enzyme present in rice. Although UGT91 D2 of the stevia plant has an activity of converting Reb A,

a steviol glycoside, to Reb D, the conversion activity is low, making it difficult to produce high concentrations of Reb M in high yield.

In addition, conventionally known UGT enzymes include UGT (hereinafter referred to as SrUGT) derived from the stevia plant (*Stevia rebaudiana*), UGT (hereinafter referred to as EUGT11) derived from rice (*Oryza sativa*), and UGT (hereinafter referred to as HvUGT) derived from barley (*Hordeum vulgare*). The UDP-glucosyltransferase of the present invention has advantages of substrate specificity that acts on several substrates of steviol glycosides and no production of reaction by-products, compared to the conventionally known EUGT11 and HvUGT. The amino acid sequence of EUGT11 enzyme is shown in SEQ ID NO: 12, and the polynucleotide sequence is shown in SEQ ID NO: 13. The amino acid sequence of HvUGT enzyme is shown in SEQ ID NO: 14, and the polynucleotide sequence is shown in SEQ ID NO: 15.

As a result of analyzing sequence identity of amino acid sequence with HvUGT and EUGT11, the wild-type enzyme having an amino acid sequence of SEQ ID NO: 1 has 91% of amino acid sequence identity with that of HvUGT and 66% with that of EUGT 11, and 36% of a polynucleotide sequence identity with HvUGT and 37% with that of EUGT11 as a result of analyzing polynucleotide sequence identity.

The UDP-glucosyltransferase according to the present invention can carry out the reaction in an aqueous system at a reaction temperature of 10 to 50°C and pH of 5.0 to 9.0. Preferably, the reaction may be performed in an aqueous system at a temperature of 25 to 40°C and a pH of 6.0 to 8.0, and more preferably, in an aqueous system at a temperature of 30°C and pH 7.2. According to a preferred example of the invention, the reaction can be performed in a phosphate buffer solution at pH 7.2.

An embodiment of the present invention relates to a nucleic acid molecule encoding a glycosyltransferase protein that transfers glucose to steviol glycosides, a recombinant vector and a transformed microorganism containing the nucleic acid molecule.

The glycosyltransferase protein that transfers glucose to the steviol glycoside can be provided as a nucleic acid molecule encoding a fusion protein to be expressed as a fusion protein fused with the sucrose synthase (SUS) enzyme capable of producing UDP-glucose. Sucrose synthase can be connected to the UDP-glycosyltransferase directly or through a linker. The glycosyltransferase protein transferring glucose, the SUS enzyme, and their fusion proteins are as described above.

According to the present invention, the recombinant microorganism or cells can be microorganisms or their cells, preferably E. coli, Saccharomyces genus strains, (e.g., Saccharomyces cerevisiae) or Pichia genus strains, but is not limited to.

As used herein, the term "transformation" refers to introducing a vector containing a nucleic acid molecule encoding a target protein into a host cell so that the protein encoded by the nucleic acid molecule can be expressed in the host cell. As long as the transformed nucleic acid molecule can be expressed in the host cell, it can include both of these molecules, regardless of whether they are inserted into the chromosome of the host cell or located outside the chromosome. Additionally, the nucleic acid molecule includes DNA and RNA that encode the target protein. The nucleic acid molecule may be introduced in any form, as long as it can be introduced into a host cell and expressed. For example, the nucleic acid molecule can be introduced into the host cell in the form of an expression cassette, which is a genetic structure containing all elements necessary for self-expression. The expression cassette may typically include a promoter, a transcription termination signal, a ribosome binding site, and a translation termination signal that are operably linked to the nucleic acid molecule. The expression cassette may be in the form of an expression vector capable of self-replication. In addition, the nucleic acid molecule may be introduced into the host cell in its own form and operably linked to the nucleic acid sequence required for expression in the host cell, but it is not limited thereto.

The vector used in this application is not particularly limited, and any vector known in the art can be used. Examples of commonly used vectors include plasmids, cosmids, viruses, and bacteriophages in a natural or recombinant state. A polynucleotide encoding a target polypeptide can be inserted into a chromosome through a vector for intracellular chromosome insertion. Insertion of the polynucleotide into the chromosome may be accomplished by any method known in the art, for example, homologous recombination, but is not limited thereto. A selection marker may be additionally included to confirm whether the chromosome has been inserted.

In addition, the term "operably linked" as used herein means that the gene sequence is functionally linked to a promoter sequence that initiates and mediates transcription of the nucleic acid molecule encoding the target protein of the present invention.

An example of a recombinant vector containing a nucleic acid molecule encoding a glycosyltransferase protein that transfers glucose to steviol glycosides according to an example of the present invention is shown in the cleavage map of FIG. 1. For example, it may include a nucleic acid molecule encoding the enzyme according to the present invention, and a GAL10 promoter and CYC1 terminator as a transcriptional regulatory sequence that can be operably linked to the nucleic acid molecule. The transcriptional promoter may include GAL10, GAL1, GAL2, TEF1, GPD1, TDH3 promoters and the like, and the transcriptional terminator may include CYC1, TEF1, PGK1, and the like.

The method of transforming the vector of the present invention includes any method of introducing a nucleic

acid molecule into a cell, and can be performed by selecting an appropriate standard technique as known in the art depending on the host cell. For example, electroporation, calcium phosphate (CaPO4) precipitation, calcium chloride (CaCl2) precipitation, microinjection, polyethylene glycol (PEG) method, DEAE-dextran method, cationic liposome method, and lithium acetate. -DMSO method, etc. can be used, but not limited to.

An embodiment of the present invention provides a composition for producing steviol glycosides, comprising at least a UDP-glucoslyltransferase enzyme protein, a recombinant microorganism expressing the enzyme protein, cells of the microorganism, lysates of the microorganism, cultures of the microorganism, and extracts thereof.

The composition for producing the steviol glycoside may further include a substrate containing at least one selected from the group consisting of stevioside and Reb A, for example, stevioside or Reb A as a single substrate, or mixed substrates. The mixed substrates may be a mixture of stevioside or Reb A, or a stevia extract (or stevia) containing stevioside or Reb A. The substrate or the raw reaction material can include Rebaudioside A in an amount of 50 to 70 (w/w)%, 50 to 65 (w/w)%, 50 to 62 (w/w)%, 55 to 70 (w/w)%, 55 to 65 (w/w)%, 55 to 62 (w/w)%, 58 to 70 (w/w) %, 58 to 65 (w/w)%, 58 to 62 (w/w)%, such as 60 (w/w)%, based on the total volume of stevia extract, and stevioside in an amount of 30 to 50 (v/v)%, 30 to 45 (v/v)%, 30 to 42 (v/v)%, 35 to 50 (v/v)%, 35 to 45 (v/v)%, 35 to 42 (v/v)%, 38 to 50 (v/v)%, 38 to 45 (v/v)% or 38 to 42 (v/v)%, such as 40 (v/v)% based on the total volume of stevia extract, but not limited thereto.

The composition for producing the steviol glycoside may further include an enzyme that converts Reb D or Reb E to Reb M, for example, a second UDP-glycosyltransferase, specifically UGT76G1 derived from stevia. The composition may further include a glucose donor, for example UDP-glucose or a saccharide capable of producing UDP-glucose.

The composition may further include Sucrose Synthase (SUS) enzyme, which can produce UDP-glucose as an example of a glucose donor, and sugar (sucrose) as a substrate of sucrose synthase. In one example of the present invention, the glycosyltransferase protein that transfers glucose to the steviol glycoside may be provided as a fusion protein with the SUS enzyme protein. In this case, the steviol glycoside can be produced without the need for the SUS enzyme protein and sugar.

The culture contains an enzyme produced from a microorganism that produces the UDP-glucosyltransferase, and may contain the microbial cells or cell-free form not containing the microbial cells. Unless otherwise specified in the present specification, it means that the used microorganisms producing UDP-glucosyltransferase include cells of the microorganism, cultures of the microorganism, lysates of the microorganism, supernatant of the lysate, and extracts thereof.

The culture of microorganisms contains an enzyme produced from a microorganism that produces the UDP-glucosyltransferase, and may contain the microbial cells or cell-free form not containing the microbial cells. Unless otherwise specified in the present specification, it means to include at least one selected from the group consisting of cells of the used microorganisms producing UDP-glucosyltransferase, cultures of the microorganism, lysates of the microorganism, supernatant of the lysates, and extracts thereof.

When steviol glycosides are produced by using the composition for producing steviol glycosides, the reaction temperature and reaction pH conditions using UDP-glucosyl transferase or a microorganism producing the enzyme are described in detail in the reaction temperature and reaction pH conditions of the enzyme.

An embodiment of the present invention is a method of producing steviol glycosides, including a step of reacting at least one selected from the group consisting of a glycosyltransferase protein that transfers glucose to the steviol glycoside, a recombinant microorganism expressing the enzyme protein, cells of the microorganism, lysates of the microorganism, cultures of the microorganism, and extracts thereof, with the substrate of the enzyme.

The prepared steviol glycoside may be at least one selected from the group consisting of Reb D and Reb E, or may be Reb M.

In an example of the present invention, when the prepared steviol glycoside is at least one selected from the group consisting of Reb D and Reb E, it provide a method of producing at least one selected from the group consisting of Reb D and Reb E which includes a step of reacting at least one selected from the group consisting UDP-glucosyltransferase protein according to the present invention, a recombinant microorganism expressing the enzyme protein, cells of the above-mentioned microorganisms, lysates of the above-mentioned microorganisms, cultures of the above-mentioned microorganisms, and extracts thereof, with at least one substrate selected from the group consisting of stevioside and Reb A, in the presence of a glucose donor.

In an example of the present invention, when the prepared steviol glycoside is Reb M, it provides a method of producing Reb M which includes.

A step of producing at least one selected from the group consisting of Reb D and Reb E by reacting at least one selected from the group consisting UDP-glucosyltransferase protein according to the present invention, a recombinant microorganism expressing the enzyme protein, cells of the above-mentioned microorganisms, lysates of the above-mentioned microorganisms, cultures of the above-mentioned microorganisms and extracts thereof, with at least one substrate selected from the group consisting of stevioside and Reb A, in the presence of a glucose donor, and
a step of reacting the prepared steviol glycosides with at least one selected from the group consisting of the second

UDP-glycosyltransferase enzyme protein, a recombinant microorganism expressing the enzyme protein, cells of the above-mentioned microorganisms, lysates of the above-mentioned microorganisms, cultures of the above-mentioned microorganisms, and extracts thereof.

The second UDP-glucosyltransferase protein used in the production step of Reb M may be UGT76G1 or the like.

In the step of preparing the steviol glycoside, the glucose donor provided may be UDP-glucose, which may be provided as a compound or may be provided as a sugar and sucrose synthase (SUS) enzyme. SUS enzyme is an enzyme that decomposes sugar to produce fructose and UDP-glucose. Therefore, by supplying UDP-glucose to the UGT enzyme with introducing SUS enzyme into the reaction for converting Reb A to Reb D, or converting stevioside to Reb E, the reaction can be performed with supplying UDP-glucose more economically. In addition, if the conversion reaction is performed by preparing SUS as a fusion protein to UGT, the two reactions can form one channel and the single enzymatic reaction can occur, resulting in an efficient conversion reaction. The SUS enzyme and the fusion protein of UGT and SUS are as described above.

The method for producing steviol glycosides according to the present invention can be economically used by the food and beverage industries, because it can significantly shorten the production cycle, improve production capacity, and provide products with lower costs and higher purity.

The substrate of UDP-glucosyltransferase according to the present invention can be a stevia extract. The stevia extract may be a substrate containing 50% of Reb A and 90% of the proportion of steviol glycosides in the extract. For example, enzymatic conversion can be performed using a stevia extract from SINOCHEM (China), which contains 50% of Reb A and contains 90% of steviol glycosides.

The steviol glycosides produced using the UDP-glucosyltransferase according to the present invention and a recombinant microorganism expressing the enzyme protein are a high-intensity sweetener that is used as an additive to various foods, beverages, etc. or are sold alone as a table sweetener.

[ADVANTAGEOUS EFFECTS]

The UDP-glucosyltransferase according to the present invention has advantages of substrate specificity that acts on various substrates of steviol glycosides and no production of reaction by-products compared to conventionally known UGTs. At least one selected from the group consisting of the enzyme protein, a recombinant microorganism expressing the enzyme protein, cells of the microorganism, lysates of the microorganism, cultures of the microorganism, and extracts thereof, can be reacted with at least one substrate selected from the group consisting of stevioside and Reb A in the presence of a glucose donor. The prepared steviol glycosides can be used by adding them to various foods, beverages, and the like, as a high-intensity sweetener.

[BRIEF DESCRIPTION OF THE DRAWINGS]

Fig. 1 is a cleavage map of a vector for preparing a yeast strain producing an enzyme converting to Reb D according to an example of the present invention.

Fig. 2 shows a graph of the results obtained by analyzing the reaction product of an enzyme converting to Reb D according to an example of the present invention by HPLC analysis.

[MODE FOR INVENTION]

The present invention will be explained in more detail through the following examples, but it is not intended that the scope of the invention be limited to the following examples.

**Example 1. Production of microorganisms expressing wild-type enzyme**

1-1: Construction of vector expressing wild-type enzyme

A gene encoding a wild-type enzyme protein having an amino acid sequence of SEQ ID NO: 1 derived from *Triticum aestivum* was synthesized (Gblock synthesis). The polynucleotide sequence of the synthesized gene is shown in SEQ ID NO: 2. The synthesized gene was subcloned into a plasmid expressed in S. *cerevisiae* to produce a form capable of gene expression.

Specifically, the vector prepared to test protein activity was pRS426 vector containing a Ura auxotrophic marker (selection marker) that could be selected in S. cerevisiae, the GAL10 promoter was used as the promoter, and CYC1 was used as a terminator. The newly synthesized gene of SEQ ID NO: 2 (IDT, gBlock) was cloned into the plasmid. For

cloning TaUGT gene, a pair of primers (SEQ ID NO: 19 and SEQ ID NO: 19) was synthesized to overlap with GAL10 promoter (SEQ ID NO: 16) and cyc1 terminator(SEQ ID NO: 17), and the enzyme gene was amplified through PCR amplification and was subcloned through Gibson assembly (HIFi DNA master mix, NEW ENGLAND BIOLABS). The gene in the form of a subcloned plasmid was selected and amplified by being transformed to into *E. coli* (DH5a), and the sequence of the amplified plasmid was analyzed to identify that the gene expressing the enzyme was accurately subcloned.

[Table 1]

| Name | Polynucleotide sequence (5'->3') | SEQ ID NO |
|---|---|---|
| Reverse primer of GAL10 promoter | catCAATTCttactttttttttggatgg | 16 |
| Forward primer of CYC1 terminator | TGATTGTCGATATCATGTAATTAGT TATGTC | 17 |
| Forward primer of TaUGT connecting with GAL10 promoter | catccaaaaaaaaagtaaGAATTGATGG ACGACGGGTCTTCTAG | 18 |
| Reverse primer of TaUGT connecting with CYC1 terminator | CTAATTACATGATATCGACAATCAT TCTTTATAGGACCTTAGCTGttg | 19 |
| Forward primer of EUGT11 connecting | catccaaaaaaaaagtaaGAATTGATGG | 20 |
| with GAL10 promoter | ACTCCGGCTACAGTTC | |
| Reverse primer of EUGT11 connecting with CYC1 terminator | CTAATTACATGATATCGACAATTAG TCCTTATAAGAACGTAGCTG | 21 |
| Forward primer of HvUGT connecting with GAL10 promoter | catccaaaaaaaaagtaaGAATTGATGG ACGGCGACGGAAAC | 22 |
| Reverse primer of HvUGT connecting with CYC1 terminator | CTAATTACATGATATCGACAAtcaA GCTTTGTAGGAACGCAGTTG | 23 |

The transformation for introducing the gene into S. cerevisiae CENPK2-1c (Euroscarf) was performed through heat shock by treating with 0.1M LiAc (Lithium Acetate). The transformed strain was selected on SC_Ura medium, and the selected colonies were cultured to test enzyme activity.

The prepared recombinant plasmid was transformed into S. cerevisiae CENPK2-1c (Euroscarf) strain. Recombinant strains with the inserted gene cassette were selected using a selection marker on SC-Ura solid medium (SC_Ura medium + 2% agar). The composition of SC-Ura solid medium included YNB 6.7 g/L, Drop-out mix 0.7 g/L, Glucose 50 g/L, and agar 20g/L, and the pH was adjusted to 6.0.

1-2: Culture of the recombinant microorganisms

The yeast transformed with the plasmid prepared in Example 1-1 was selected in SC (synthetic complete) medium using URA-drop out mix plate, and was induced to express enzyme through liquid culture.

Specifically, colonies selected from SC_Ura solid medium were inoculated into a test tube containing 3 mL SC_Ura liquid medium and were cultured overnight. The concentration of the cultured cells was measured with $OD_{600}$ absorbance, and the cultured cells were inoculated in a 250 mL flask containing 25 mL of SC-Ura liquid medium until the $OD_{600}$ absorbance was 0.1 (final), and incubated for 24 hours (30°C, 240 rpm). To induce enzyme expression in the primary cultured cells, the cells were cultured secondly for 24 hours with adding an equal amount of YPG medium. The secondary cultured cells were collected by centrifugation (4,000 rpm, 10 min). The composition of SC_Ura liquid medium included YNB (yeast nitrogene base) 6.7 g/L, Drop-out mix /L, Glucose g/L, and MES mM, and the medium pH was adjusted to 6.0 using NaOH. The YPG medium composition included 20 g/L of Bacto peptone, 10 g/L of Yeast

Extract, 20 g/L of Galactose, and 100 mM Phosphate buffer (sodium salt), and the pH of the medium was adjusted to 6.0.

1-3: Production of microorganisms expressing EUGT11 and HvUGT

The EUGT11 gene (SEQ ID NO: 13) from *Oryza sativa,* which has known to its activity, and the HvUGT gene (SEQ ID NO: 15) from *Hordeum vulgare* were synthesized (Gblock synthesis), and the genes were amplified in the same manner as the TaUGT gene and cloned into the pRS426 vector. To induce enzyme expression under the same conditions, the GAL10 promoter was used as the promoter and CYC1 as the terminator. Specifically, the forward primer of EUGT11 linked to the GAL10 promoter (SEQ ID NO: 20), the reverse primer of EUGT11 linked to the CYC1 terminator (SEQ ID NO: 21), the forward primer of HvUGT linked to the GAL10 promoter (SEQ ID NO: 22), and the reverse primer of HvUGT linked to the CYC1 terminator (SEQ ID NO: 23) were used. The gene cassette for producing enzyme was shown in Table 1 above, and Sc refers to *Saccharomyces cerevisiae.*

Thereafter, transformation of S. cerevisiae CENPK2-1c (Euroscarf), strain selection and microbial culture were performed using substantially the same method as in Examples 1-1 and 1-2.

1-4: Evaluation of enzyme activity

The cells were collected from the cultures of the recombinant strains obtained in Examples 1-2 and 1-3 by centrifugation (4,000 rpm, 10 min). 10 mL of the collected cells were washed twice with the same amount of water. The washed microbial cells were resuspended in 100 mM phosphate buffer (pH 7.2) so that the $OD_{600}$ value was 100. Then, 100 mg of glass beads (Sigma) with a particle diameter of 0.4 to 0.6 mm were placed in a cap tube, and added by 1ml of 100 mM phosphate buffer (pH 7.2). The cells were disrupted five times for 30 seconds in a bead beater. The cell lysate liquid was centrifuged (13,000 rpm, 15 min) in refrigerated condition to obtain a supernatant as a crude enzyme solution.

The reaction solution for evaluating enzyme activity contained the substrate of Reb A (95%, Sinochem), $MgCl_2$, and UDP-Glucose. In addition, 0.1 mL of the crude enzyme solution was added to prepare a total reaction volume of 0.5 mL, and the final reaction solution was adjusted to 2 g/L of Reb A (95%, Sinochem), 3mM of $MgCl_2$, and 4 mM of UDP-Glucose. The enzyme reaction was performed on the prepared reaction solution at a temperature of 30°C, pH 7.2, and 150 rpm, and samples were taken at 3 hours and 18 hours after the start of the reaction to check the degree of reaction according to the rebound time.

The enzyme reaction solution was boiled for 5 minutes to terminate the enzyme reaction and centrifuged (13,000 rpm, 10 min). The obtained supernatant was analyzed to analyze the enzyme reaction product. Specifically, the enzyme reaction product was analyzed using HPLC Chromatography to measure the conversion rate of the product. Since the crude enzyme solution obtained from the cell lysate of the recombinant strain showed the activity of converting Reb A substrate to Reb D, the activity was measured by using the production ratio of Reb D.

Specifically, the column used for HPLC analysis of the conversion product was UG120 (C18 250 mm × 46 mm, 5 um 110 A particle, Shideido), and the analysis was performed at 210 nm. The mobile phase was used in a gradient with water containing 0.01% TFA (Trifluoroacetic acid, Sigma) and acetonitrile, respectively. The mobile phase was flowed at 1 mL/min and analyzed for a total of 40 minutes. The HPLC analysis conditions are shown in Table 2 below.

[Table 2]

| Time (min) | Eluent A(%) | Eluent B(%) |
|---|---|---|
| 0 | 80 | 20 |
| 10 | 80 | 20 |
| 12 | 65 | 35 |
| 30 | 0 | 100 |
| 32 | 80 | 20 |
| 40 | 80 | 20 |

The HPLC graph of the conversion product is shown in Table 3. The Reb D conversion rate of the enzyme that converts Reb A as a single substrate to Red D shown in Table 3 below is calculated according to Formula 1 below, the relative Reb D conversion rate of the enzyme refers to the Reb D conversion rate of TaUGT or HvUGT enzyme calculated based on 100% Reb D conversion rate of EUGT11.

[Formula 1]

Reb D conversion rate (%) = HPLC peak area of Reb D / Total HPLC peak area

[Table 3]

| Enzyme name | Reaction time (hour) | Conversion rate of Reb D (%) | Relative conversion rate of Reb D (%) |
|---|---|---|---|
| EUGT11 | 3 | 45.8 | 100% |
| TaUGT | 3 | 43.4 | 94.8% |
| HvUGT | 3 | 32.5 | 71.0% |
| EUGT11 | 18 | 70.6 | 100% |
| TaUGT | 18 | 68.0 | 96.3% |
| HvUGT | 18 | 62.2 | 88.1% |

As shown in Table 3, the conversion rate of TaUGT in enzymatic reaction was similar to EUGT11 as a result of the initial 3-hour reaction that TaUGT converted 40-45% of Reb A to Reb D, and had an improved activity of about 30% higher than HvUGT (30-35% conversion).

In addition, as a result of analyzing the Reb D content produced in the 18-hour enzyme reaction solution, the order of conversion rates of the three enzymes showed a similar tendency to those of the 3-hour reaction.

The polynucleotide sequence of TaUGT enzyme was analyzed through the polynucleotide sequence analysis service of Macrogen Co., Ltd., and the amino acid sequence was analyzed based on the polynucleotide sequence. As a result, the amino acid sequence of TaUGT enzyme is shown in SEQ ID NO: 1 and the polynucleotide sequence is shown in SEQ ID NO: 2..

**Example 2. Production of microorganisms expressing mutant enzyme**

1-1: Construction of vector expressing mutant enzyme

In order to prepare a mutant of TaUGT enzyme of Example 1, mutations were searched based on the EUGT11 gene. The conserved region was identified through sequence alignment, and the amino acid differences was analyzed at position between the domains of the N-term sugar acceptor and C-term sugar donor of UGT. Among them, at threonine at residue 201 and valine at residue 202 (corresponding to serine and leucine in EUGT11, respectively), threonine was replaced with serine, and valine was replaced with alanine or leucine. The mutation method included synthesizing primers (SEQ ID NOs. 24 and 25) at the corresponding region of TaUGT, and performing mutant gene amplification (PCR) to replace the polynucleotide sequence at the corresponding region, and cloning the amplified gene fragment into the pRS426 vector through Gibson assembly.

The resulting mutant enzyme was cloned into the pRS426 vector, which was named as TaUGT$^{T201S/V202L}$. The sequences of primers used in this example are listed in Table 4 below.

[Table 4]

| Name | Polynucleotide sequence (5'->3') | SEQ ID NO |
|---|---|---|
| Forward primer for T201S mutant or V202L mutant of TaUGT | caggtatgtcwbtagcagagcgttatttcctg | 24 |
| Reverse primer for T201S mutant or V202L mutant of TaUGT | cgctctgctavwgacatacctgatgcattctgag | 25 |

2-2: Production of microorganisms expressing mutant enzyme

In order to test the enzyme expression, TaUGT $^{T201S/V202L}$ cloned into the pRS426 vector was selected by transforming into the same yeast strain using the LiAc method in substantially the same manner as in Example 1-2. The transformed strain was cultured in substantially the same manner as in Example 1-2. Yeast transformed with the plasmid prepared in Example 2-1 was selected in SC (synthetic complete) medium using URA-drop out mix, and the enzyme expression was induced in the selected yeast through liquid culture.

**Example 3. Evaluation for the activity of mutant enzyme**

The cells were collected from the cultures of the recombinant strains obtained in Example 2-2 by centrifugation (4,000 rpm, 10 min). 10 mL of the recovered cells were collected and washed twice with the same amount of water. The washed microbial cells were resuspended in 100 mM phosphate buffer (pH 7.2) so that the $OD_{600}$ value was 100. Then, 100 mg of glass beads (Sigma) with a particle diameter of 0.4 to 0.6 mm were placed in a cap tube, and added by 1ml of 100 mM phosphate buffer (pH 7.2). The cells were disrupted five times for 30 seconds in a bead beater. The cell lysate liquid was centrifuged (13,000 rpm, 15 min) in refrigerated condition to obtain a supernatant as a crude enzyme solution.

The reaction solution for evaluating enzyme activity contained the substrate of Reb A (95%, Sinochem), $MgCl_2$, and UDP-Glucose. In addition, 0.1 mL of the crude enzyme solution was added to prepare a total reaction volume of 0.5 mL, and the final reaction solution was adjusted to 2 g/L of Reb A (95%, Sinochem), 3mM of $MgCl_2$, and 4 mM of UDP-Glucose. The enzyme reaction was performed on the prepared reaction solution at a temperature of 30°C, pH 7.2, and 150 rpm, and samples were taked at 3 hours and 18 hours after the start of the reaction to check the degree of reaction according to the rebound time.

The enzyme reaction solution was boiled for 5 minutes to terminate the enzyme reaction and centrifuged (13,000 rpm, 10 min).The obtained supernatant was analyzed to analyze the enzyme reaction product. Specifically, the enzyme reaction product was analyzed using HPLC-Chromatography, to measure the conversion rate of the product. Since the crude enzyme solution obtained from the cell lysate of the recombinant strain showed the activity of converting Reb A substrate to Reb D, the activity was measured by using the production ratio of Reb D.

Specifically, HPLC analysis of the conversion product was performed at 210 nm using UG120 ($C_{18}$ 250 mm × 46 mm, 5 um 110 A particle, Shiseido) as a column. The mobile phase was water containing 0.01% TFA (Trifluoroacetic acid, Sigma) and acetonitrile, respectively, and was used as a concentration gradient. The mobile phase was flowed at 1 mL/min and confirmed through analysis for a total of 40 minutes. The HPLC analysis conditions are shown in Table 4 below. A graph of the HPLC analysis of the conversion product is shown in Fig. 2. The Reb D conversion rate of the enzyme converting Reb A as a single substrate to Red D shown in Table 4 below, is calculated according to Formula 1 below, and the relative Reb D conversion rate of the enzyme refers to the Reb D conversion rate of TaUGT mutant enzyme, which is converted based on 100% of Reb D conversion rate of the wild type enzyme.

[Formula 1]

$$\text{Reb D Conversion rate (\%)} = \text{HPLC peak area of Reb D} / \text{Total HPLC peak area}$$

An enzymatic reaction was performed for 18 hours using the reaction solution containing the mutant enzyme of TaUGT and Reb A as a single substrate, and the obtained conversion product was analyzed by HPLC analysis. The results of the activity analysis of TaUGT mutant enzymes using the HPLC analysis method are shown in Table 5 below and Fig. 2. Fig. 2 is an HPLC graph to analyze the reaction product of Reb D conversion reaction using a mutant enzyme of TaUGT.

[Table 5]

| Enzyme name | Conversion rate of Reb D(%) | Relative conversion rate of Reb D (%) |
|---|---|---|
| TaUGT (wild type) | 68.0 | 100% |
| T201S | 75.5 | 110.3% |
| T201S/V202L | 86.3 | 129.1% |

**Example 4. Conversion reaction using mixed substrates**

The cells of the recombinant strain were resuspended in 100 mM phosphate buffer (pH 7.2) so that the OD600 value was 100. 100 mg of glass beads (Sigma) with a particle diameter of 0.4 to 0.6 mm were placed in a cap tube, and 1 ml of 100 mM phosphate buffer (pH 7.2) was added. The cells were disrupted five times for 30 seconds in a bead beater. The cell lysate liquid was centrifuged (13,000 rpm, 15 min) in refrigerated condition to obtain a supernatant, which was used as a crude enzyme solution.

The reaction solution for evaluating enzyme activity contained the reaction substrates of RA40 (Reb A (rebaudioside A: STE (stevioside) = 40:60, Sinochem), $MgCl_2$, and UDP-Glucose. In addition, 0.2 mL of the crude enzyme

solution of Example 3 was added to prepare a total reaction volume of 0.5 mL, and the final reaction solution was adjusted to 2 g/L of RA40 (Reb A (rebaudioside A: STE (stevioside) = 40:60, Sinochem), 3mM of $MgCl_2$, and 4 mM of UDP-Glucose. The enzyme reaction was performed on the prepared reaction solution at a temperature of 30°C, pH 7.2, and 150 rpm, and the degree of reaction was checked at 18 hours after reaction and samples were taken at 3 hours and 18 hours after the start of the reaction to check the degree of reaction according to the rebound time. The conversion product was analyzed with HPLC analysis method in substantially same method of Example 3.

The total Reb D/E conversion rate of the enzyme converting RA40 mixed substrates to Red D/E shown in Table 6 below, is calculated according to Formula 2 below, and the relative total Reb D/E conversion rate of the enzyme refers to the total Reb D/E conversion rate of TaUGT enzyme, which is converted based on 100% of total Reb D/E conversion rate of EUGT11 enzyme.

Reb D/E conversion rate (%) = HPLC peak area of Reb D/E/Total HPLC peak area [Formula 2]

[Table 6]

| Enzyme name | Reaction time (hour) | Total conversion rate of Reb D/E(%) | Total relative conversion rate of Reb D/E (%) |
|---|---|---|---|
| TaUGT (wild type) | 18 | 78.0 | 100 |
| TaUGT (T201S) | 18 | 83.0 | 106 |
| TaUGT (T201 SV202L) | 18 | 89.2 | 114 |

As a result of the above experiment, the mutant enzyme in which Threonine at 201 residue was replaced with Serine had a relative conversion rate of 106% based on the wild type enzyme, and the double mutant enzyme containing both the substitution of Serine for Threonine at 201 residue and the substitution of Leucine for Valine at 202 residue had increased to 114 % of relative conversion rate based on the wild-type enzyme. Accordingly, the residues whose activity was improved through mutation were identified and the mutant enzymes with increased activity were obtained.

**Example 5. Preparation of fusion enzyme of TaUGT_SUS and its activity evaluation**

In order to supply the glucose donor (UDP-Glucose) required to perform the glycotransferase reaction of steviol glycosides using the TaUGT_T201SV202L (TaUGTm) mutant enzyme which was obtained to have improved activity prepared in Example 3, the mutant enzyme was prepared as a fusion protein with sucrose synthase (SUS1, derived from *Arabidopsis thaliana*).

The nucleotide sequence encoding the TaUGT_T201SV202L (TaUGTm) mutant enzyme was the nucleotide sequence of SEQ ID NO: 30 in Table 7 below, and the nucleotide sequence encoding the sucrose synthase (SUS1, derived from *Arabidopsis thaliana*) was the nucleotide sequence of SEQ ID No: 5 in Table 7.

[Table 7]

| Name | sequence (5' -> 3') | SEQ ID NO |
|---|---|---|
| TaUGTm_Protei n | MDDGSSSSSSPLRVVICPWLAFGHLLPCLDIAERLASRGHRVSFVS TPRNIARLPPVRPAVAPLVDYVALPLPRVDGLPEGAESTNDVPHDKF ELLRKAFDGLAAPFSEFLHAACAEGTGKRPDWLIVDSFHHWAAAAA VENKVPCVMLLLGAANVIATWARGVSEHAAAAVGKERSAAEAPSFE TERRKLMITQNASGMSLAERYFLTLMRSNLVAIRSCAEWEPESVAAL TTLAGKPVVTLGLLPPSPEGGRGISKQDAAVRWLDAQRDKSVVYVA LGSEVPLRVEQVHELALGLELSGASFLWALRKPPGMPDAAVLPPGF EERTRGRGLVVTGWVPQISVLAHGAVAAFLTHCGWNSTIEGLLFGQ PLIMLPISSDQGPNARLMEGRKVGMQVPRNESDGSFTREDVAATV QAVAMEEDGSRVFTANAKTMQEIVADSACHERCIDGFIQQLRSYKE | 3 |

(continued)

| Name | sequence (5’ -> 3’) | SEQ ID NO |
|---|---|---|
| TaUGTm_DNA | ATGGACGACGGGTCTTCTAGCTCTAGCAGTCCGCTGCGTGTTGTTATTTGCCCGTGGCTGGCTTTTGGACACCTGCTTCCATGCTTAGACATTGCAGAAAGATTAGCCAGCCGTGGACACAGAGTATCCTTCGTAAGTACGCCGCGTAATATAGCACGTTTACCCCCGGTCAGACCGGCAGTTGCGCCGCTGGTCGATTACGTAGCGCTACCTCTGCCCAGAGTGGACGGACTTCCGGAAGGGGCTGAATCTACAAACGACGTGCCCCATGATAAGTTCGAGCTTTTACGTAAGGCTTTCGACGGTTTGGCAGCGCCCTTCAGCGAATTTCTACACGCAGCGTGCGCGGAGGGCACAGGCAAAAGACCAGATTGGCTTATTGTGGATTCCTTCCACCATTGGGCTGCGGCTGCGGCAGTGGAAAATAAAGTCCCGTGCGTCATGTTACTTTTAGGTGCAGCCAACGTGATCGCCACTTGGGCACGTGGCGTGTCAGAGCATGCGGCCGCCGCGGTTGGAAAAGAACGTAGTGCCGCAGAAGCTCCATCATTCGAAACCGAACGTCGTAAACTGATGATAACTCAGAATGCATCAGGTATGTCATTAGCAGAGCGTTATTTCCTGACACTTATGAGATCAAACTTGGTTGCCATTCGTTCCTGTGCAGAATGGGAGCCTGAATCTGTGGCGGCACTAACCACTCTGGCCGGGAAGCCCGTAGTGACCTTAGGTTTGCTTCCACCGTCTCCTGAGGGAGGAAGGGGTATTTCCAAGCAAGACGCTGCCGTTAGGTGGCTGGACGCTCAGAGGGATAAGTCCGTTGTATACGTGGCGTTAGGGTCTGAAGTTCCCTTGAGAGTGGAGCAGGTACATGAGCTTGCGCTTGGACTAGAGTTGTCAGGTGCTAGCTTCCTGTGGGCGTTACGTAAGCCACCCGGCATGCCTGACGCCGCAGTCTTGCCGCCGGGTTTTGAAGAGAGAACCAGGGGACGTGGTCTGGTTGTGACTGGCTGGGTACCACAAATCAGCGTCTTAGCACATGGCGCCGTTGCCGCTTTTCTGACACATTGCGGCTGGAATAGTACAATCGAAGGCTTGCTGTTCGGACAACCGTTAATCATGCTGCCAATCAGTAGCGATCAGGGCCCGAATGCCCGTCTTATGGAGGGAAGAAAAGTTGGAATGCAGGTGCCTCGTAATGAGTCCGATGGATCATTCACAAGAGAGGATGTCGCTGCCACAGTACAAGCAGTAGCGATGGAGGAGGATGGGAGCCGTGTTTTTACGGCTAATGCAAAGACCATGCAAGAAATAGTTGCTGACTCCGCGTGTCACGAAAGGTGCATCGATGGATTTATCCAACAGCTAAGGTCCTATAAAGAA | 30 |
| Sucrose_syntha se_ Protein | MANAERMITRVHSQRERLNETLVSERNEVLALLSRVEAKGKGILQQNQIIAEFEALPEQTRKKLEGGPFFDLLKSTQEAIVLPPWVALAVRPR | 4 |

(continued)

| Name | sequence (5' -> 3') | SEQ ID NO |
|---|---|---|
| derived from Arabidopsis thaliana | PGVWEYLRVNLHALVVEELQPAEFLHFKEELVDGVKNGNFTLELDFEPFNASIPRPTLHKYIGNGVDFLNRHLSAKLFHDKESLLPLLKFLRLHSHQGKNLMLSEKIQNLNTLQHTLRKAEEYLAELKSETLYEEFEAKFEEIGLERGWGDNAERVLDMIRLLLDLLEAPDPCTLETFLGRVPMVFNVVILSPHGYFAQDNVLGYPDTGGQVVYILDQVRALEIEMLQRIKQQGLNIKPRILILTRLLPDAVGTTCGERLERVYDSEYCDILRVPFRTEKGIVRKWISRFEVWPYLETYTEDAAVELSKELNGKPDLIIGNYSDGNLVASLLAHKLGVTQCTIAHALEKTKYPDSDIYWKKLDDKYHFSCQFTADIFAMNHTDFIITSTFQEIAGSKETVGQYESHTAFTLPGLYRVVHGIDVFDPKFNIVSPGADMSIYFPYTEEKRRLTKFHSEIEELLYSDVENKEHLCVLKDKKKPILFTMARLDRVKNLSGLVEWYGKNTRLRELANLVVVGGDRRKESKDNEEKAEMKKMYDLIEEYKLNGQFRWISSQMDRVRNGELYRYICDTKGAFVQPALYEAFGLTVVEAMTCGLPTFATCKGGPAEIIVHGKSGFHIDPYHGDQAADTLADFFTKCKEDPSHWDEISKGGLQRIEEKYTWQIYSQRLLTLTGVYGFWKHVSNLDRLEARRYLEMFYALKYRPLAQAVPLAQDD | |

(continued)

| Name | sequence (5' -> 3') | SEQ ID NO |
|---|---|---|
| Sucrose_syntha se_ nucleotide sequence derived from Arabidopsis thaliana | atggctaacgccgaacgtatgatcacgagggtacactcccaacgtgaacgtttaaatgaaact<br>ttggtcagcgagcgtaacgaggttctagcactactttccagggtcgaagctaaggggaagggg<br>atacttcagcaaaatcagataatcgcggagttcgaggctttaccggagcagaccagaaaaaa<br>gttagagggtggcccgttctttgatctacttaaatctacacaggaggctattgtgctgccaccttgg<br>gtcgccctggcagtcaggcctaggccaggcgtatgggagtaccttcgtgttaatttgcacgcatt<br>agtagtggaggaattacagcctgccgaatttctacactttaaagaggagttagtggacggtgtc<br>aagaatggaaacttcacccttgagcttgatttcgaaccattcaatgccagcataccccgtcctac<br>attacacaaatatattgggaacggggtcgactttctgaataggcatctatcagctaagttgttccat<br>gacaaggaatcccttcttcctctattgaaatttttgagacttcacagtcatcaggggaagaacttg<br>atgttaagcgagaaaatacaaaacttgaatacgttacaacatacgcttagaaaggcagagga<br>gtatctggcggaactaaagagtgagactttgtacgaagagttcgaagccaagtttgaggaaat<br>aggtcttgaacgtgggtggggcgacaatgctgaacgtgtcctggatatgatacgtcttttactgga<br>cttattagaggcaccggacccatgtaccctagaaacattcttagggagggtacccatggttttca<br>acgttgtcattcttagtccgcatggatattttgcccaagataacgtgttaggataccccgacaccg<br>ggggacaagttgtttatattttagatcaagtaagagcattagagattgagatgttacaaagaatta<br>aacagcagggtctgaacataaaaccaaggatcttaattttgactcgtcttcttcctgatgcagtgg<br>ggacgacgtgcggagagaggctggaacgtgtctacgattcagaatattgtgatatcctgagag<br>tgccctttagaacagaaaaaggaatcgtgcgtaagtggatcagtagatttgaagtctggccata<br>tctggagacatacaccgaagatgcagcggtcgaacttagcaaagagctaaacgggaaacc<br>cgacctgataatcgggaactacagcgacgggaatttagttgccagtttacttgcccataagctg<br>ggagtgacacagtgcacgatagcccacgcccttgaaaagacgaagtatcccgattctgacat<br>atattggaaaaaacttgatgacaaataccatttttcatgccaattcactgctgatatctttgcaatga<br>atcacacggatttcatcattacttccacatttcaagagatcgcaggcagcaaagagacagttgg<br>acaatatgagagtcatactgcattcacactacctggtctttatagagtcgtgcacggtatagacgt<br>gtttgatcctaagttcaacatagtctcaccgggtgcggatatgtccatctactttccctacactgag<br>gaaaagaggcgtttaaccaagtttcattcagagatagaagagttattatatagtgatgtggaaa<br>ataaagagcatctatgcgtgttaaaagacaagaagaaacctatcctattcacaatggccagac<br>ttgacagagtgaagaatctatccggcttggttgaatggtacggcaagaacacgcgtcttcgtga<br>gctagcgaaccttgtcgtagtaggaggtgacagaagaaaggaatccaaagacaacgaaga<br>gaaagcggagatgaaaaagatgtacgatctaatcgaagaatacaaattaaatggtcagttcc<br>gttggatctcctctcagatggatagagtccgtaatggagaactatacagatatatatgcgacacc<br>aaaggcgcgtttgtgcagccggcattgtacgaggcctttggtctgactgtcgtcgaggccatgac<br>ctgcggccttcctacattcgcgacctgcaaaggtggtccagctgagattattgtccatgggaaat | 5 |
| | ctggattccacatagacccatatcatggtgaccaggccgctgatacacttgcagatttttttactaa<br>gtgtaaggaggacccttcacattgggatgaaatatccaaaggggggactgcagcgtatcgagg<br>agaaatatacctggcaaatatatagccaaaggctgttgaccctaacgggcgtttacgggttctg<br>gaagcacgtttccaatctagataggctagaggcgaggcgttacctggaaatgttttacgcgttga<br>aatacaggcctctagcacaagcagtaccactggcgcaggacgat | |

The preparation of the fusion enzyme was obtained by using a linker (GGGGSG, SEQ ID NO. 8) with synthesizing primers as a connecting amino acid sequence between TaUGTm and SUS, and each gene was obtained by amplifying the corresponding region according to nucleotide sequence amplification (PCR), with synthesizing primers (SEQ ID NO. 26, SEQ ID NO. 27) at the corresponding position.

After performing a gene amplification reaction (PCR), the amplified gene fragment was cloned into the pRS426 vector through Gibson assembly to produce pRS426 TaUGTm_SUS. The amino acid sequence of the SUS enzyme protein used is shown in SEQ ID NO: 4, the nucleotide sequence encoding the enzyme protein is shown in SEQ ID NO: 5, and the amino acid sequence of the fusion enzyme protein is shown in SEQ ID NO: 6. In addition, the linker sequence

used to prepare the fusion protein has GGGGSG (SEQ ID NO: 8), and the amino acid and nucleotide sequences of the SUS1 enzyme protein and information on the primers used are shown in Table 8 below.

[Table 8]

| Name | Polynucleotide Sequence (5'->3') | SEQ ID NO |
|---|---|---|
| Forward primer for TaUGT(mutant) | catccaaaaaaaaagtaagaattgatggacgacgggtcttctag | 26 |
| Reverse primer for TaUGT(mutant)_linker | tccactaccaccgccaccttctttataggaccttagctgttgg | 27 |
| Forward primer of Linker_SUS | ggtggcggtggtagtggaatggctaacgccgaacgtatg | 28 |
| Reverse primer of SUS | ctaattacatgatatcgacaattactcggcagccagagggac | 29 |

In the same manner as Example 1-2, the constructed expression vector for the fusion enzyme was transformed into yeast and selected to obtain recombinant yeast expressing the fusion enzyme.

A crude enzyme solution was prepared using the lysate of the cultured cells in substantially the same manner as in Example 1-3. Specifically, in substantially the same manner as in Example 3, the cells collected from the culture of the recombinant strain were collected using centrifugation, and the cells were crushed with glass beads and used as a crude enzyme solution.

Particularly, the raw reaction solution for conversion resection was prepared by mixing the reaction substrates of RA40 (Reb A (rebaudioside A: STE (stevioside) = 40:60, Sinochem), $MgCl_2$, UDP and sucroase, and adding 0.1 mL of the crude enzyme solution of TaUGTm_SUS fusion enzyme, to prepare a total reaction volume of 0.5 mL, and the final reaction solution was adjusted to 10 g/L of RA40 (Reb A (rebaudioside A: STE (stevioside) = 40:60, Sinochem), 1 mM of $MgCl_2$, and 1 mM of UDP, and 125 mM of sucrose. An enzyme reaction was performed on the prepared reaction solution at a temperature of 30°C, pH 7.2, and 150 rpm, and the enzyme conversion was evaluated by reacting for 20 hours.

In substantially the same way as Example 1-4, the conversion product of the reaction solution was analyzed by HPLC analysis. It was verified that the fusion enzyme converted RA40 (Reb A: STE = 40:60) to Reb D/E with supplying UDP_glucose from UDP. STE refers to stevioside.

About 33% by weight of the substrate was converted to Reb D/E through the initial conversion reaction of the fusion enzyme, on the basis of to 100% by weight of the substrate before the reaction. Through the conversion reaction of the mixed substrates (RA40 (Reb A: STE = 40:60)), Reb D (including D derivatives) and Reb E were converted to 21.7% and 12.1%, respectively. The activity of the fusion enzyme that produced UDP_Glucose from sucrose and saccharification of the substrate using the UDP_glucose were identified. It was verified that it was possible to improve the conversion reaction of the enzyme by optimizing the amount of substrate and reaction conditions.

When using a fusion enzyme to convert a substrate equivalent to about 10 mM by using 10 g/L substrate of RA40 (Reb A: STE = 40:60), more than 10 to 20 mM of UDP_Glucose is required. Therefore, when converting a substrate in a large amount at high concentration, an excess amount of expensive UDP_Glucose is required. When carrying out an enzyme reaction using a high concentration of substrate, it is not preferable, because an excessive amount of UDP_Glucose must be used and excess UDP_Glucose inhibits enzyme activity.

According to this experiment, when using the TaUGT_SUS fusion enzyme, it is possible to perform conversion using 1/10 of UDP amount for the substrate, and to perform the reaction using UDP at an amount lower than 1/100 of UDP amount for RA40 substrate. It was verified that the conversion reaction using a fusion enzyme could make an economical enzyme conversion, and that the steviol glycosides could be produced through a reaction using high concentration of substrate.

## Claims

1. An enzyme protein having a uridine diphosphate-glucosyltransferase (UDP-glucoslyl transferase) activity to transfer glucose to steviol glycoside substrate, wherein at least one amino acid selected from the group consisting of the $201^{st}$ and $202^{nd}$ amino acids from the N-terminus in the amino acid sequence of SEQ ID NO: 1 is substituted with at least one amino acid selected from the group consisting of serine and valine.

2. The enzyme protein according to claim 1, wherein the steviol glycoside substrate is at least one selected from the group consisting of stevioside and rebaudioside A.

3. The enzyme protein according to claim 1, wherein the enzyme protein has an activity of converting 45% by weight or more of Rebaudioside A into Rebaudioside D in an enzyme reaction for 2 to 24 hours.

4. The method of claim 1, wherein the enzyme protein has 101% or higher of an activity, based on 100% of an activity of the wild-type enzyme containing an amino acid sequence of SEQ ID NO: 1 for converting Rebaudioside A substrate to Rebaudioside D in an enzyme reaction for 2 to 24 hours.

5. A fusion protein in which the UDP-glucoslyltransferase according to claim 1 and a sucrose synthase are connected.

6. The fusion protein according to claim 5, wherein the sucrose synthase is a sucrose synthase derived from at least a microorganism selected from the group consisting of *Arabidopsis thaliana, Solanum lycopersicum, Glycine max, Nicotiana tabacum,* and *Thermosynechococcus elongates.*

7. A recombinant microorganism comprising a gene encoding the enzyme protein according to any one of claims 1 to 6.

8. The recombinant microorganism according to claim 7, wherein the microorganism is selected from the group consisting of *Escherichia coli, Saccharomyces* genus microorganisms, and *Pichia* genus microorganisms.

9. A composition for producing steviol glycosides, comprising at least a UDP-glucoslyltransferase enzyme protein according to any one of claims 1 to 4, a recombinant microorganism expressing the enzyme protein, cells of the microorganism, lysates of the microorganism, cultures of the microorganism, and extracts thereof.

10. The composition according to claim 9, wherein the composition further comprises at least an substrate selected from the group consisting of stevioside and rebaudioside A.

11. The composition according to claim 10, wherein the substrate is a mixture of substrates comprising stevioside and rebaudioside A.

12. The composition according to claim 9, further comprises an enzyme converting Rebaudioside D or Rebaudioside E, to Rebaudioside (Reb) M.

13. The composition according to claim 12, wherein the enzyme converting to Reb M is UGT761 derived from Stevia.

14. The composition according to claim 9, wherein the UDP-glucoslyltransferase is provided as a fusion protein in which a sucrose synthase is linked to.

15. The composition of claim 9, wherein the composition comprises a glucose donor (glycosyl donor).

16. The composition of claim 9, wherein the glucose donor is UDP-glucose.

17. The composition according to claim 9, wherein the composition comprises sucrose and UDP, when the composition further comprises a sucrose synthase, or a sucrose synthase as a fusion protein with the UDP-glucoslyltransferase.

【FIG. 1】

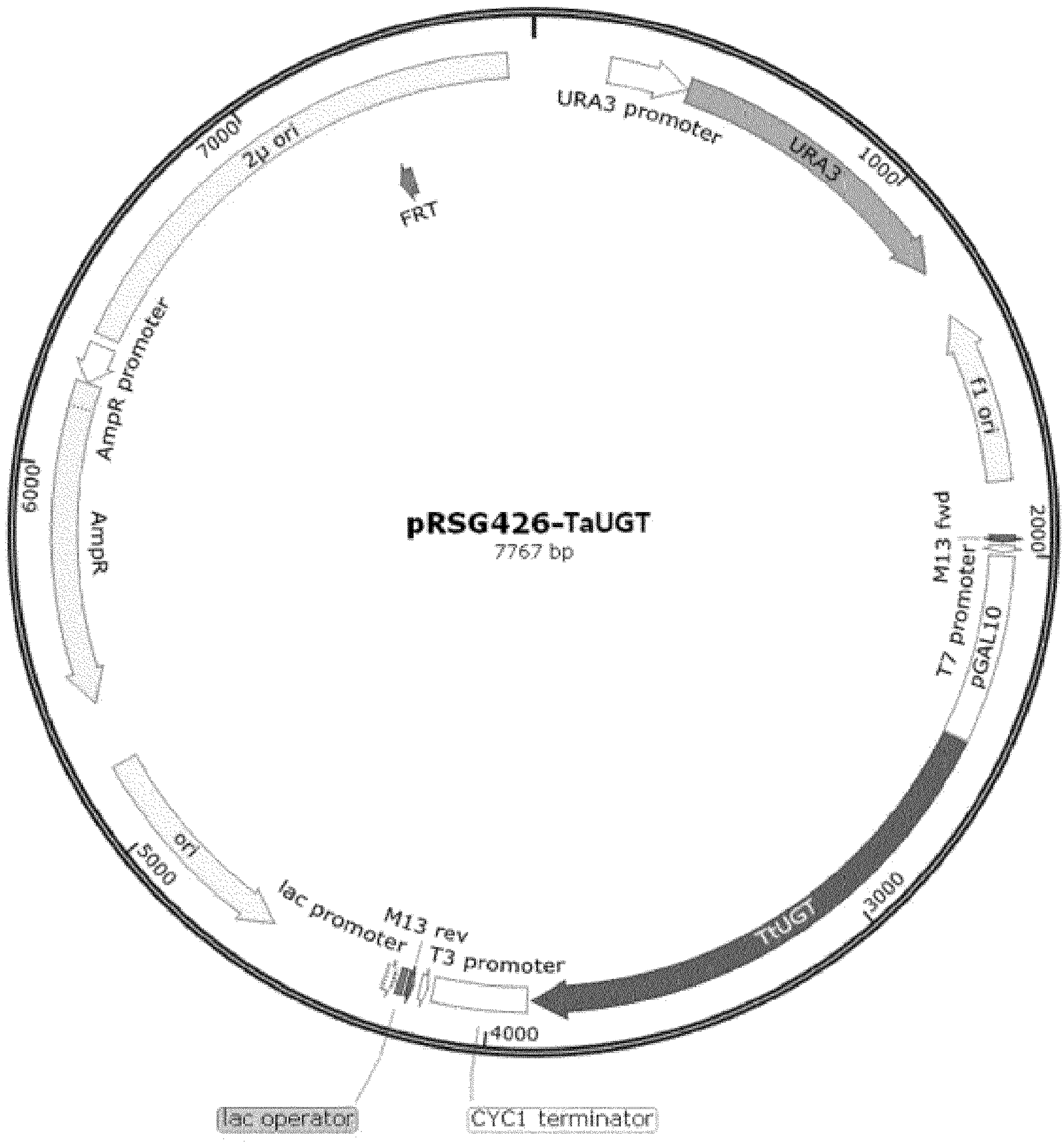
pRSG426-TaUGT
7767 bp
URA3 promoter
URA3
1000
f1 ori
M13 fwd
2000
T7 promoter
pGAL10
TtUGT
3000
T3 promoter
4000
CYC1 terminator
M13 rev
lac promoter
lac operator
ori
5000
AmpR
6000
AmpR promoter
2μ ori
7000
FRT

【FIG. 2】

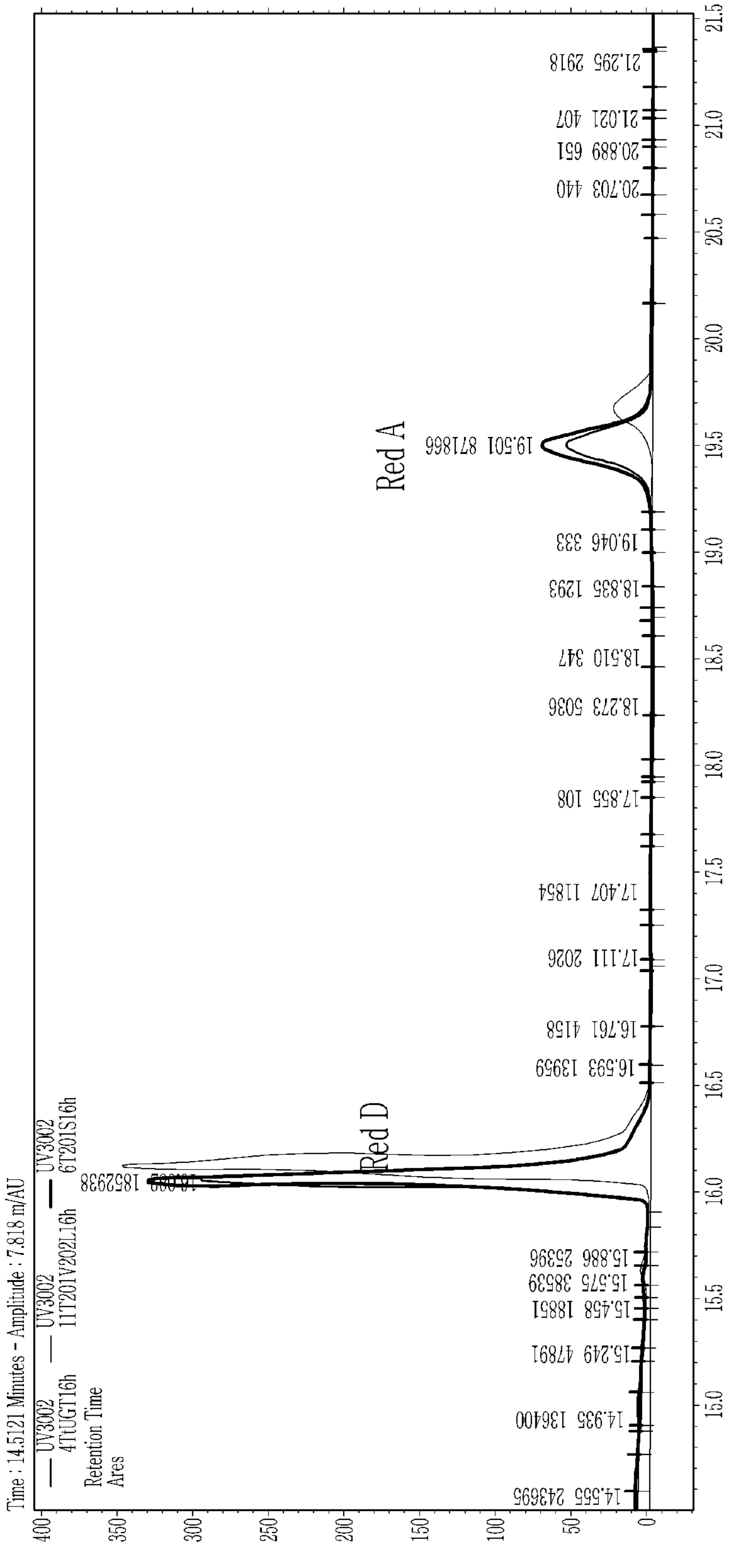

Time : 14.5121 Minutes - Amplitude : 7.818 m/AU
UV3002 4TtUGT16h
UV3002 11T201V202L16h
UV3002 6T201S16h
Retention Time
Ares
Red A
Red D
14.555 243695
14.935 136400
15.249 47891
15.458 18851
15.575 38539
15.886 25396
16.089 1852938
16.593 13959
16.761 4158
17.111 2026
17.407 11854
17.855 108
18.273 5036
18.510 347
18.835 1293
19.046 333
19.501 871866
20.703 440
20.889 651
21.021 407
21.295 2918

## INTERNATIONAL SEARCH REPORT

International application No.
**PCT/KR2022/021327**

**A. CLASSIFICATION OF SUBJECT MATTER**

**C12N 9/10**(2006.01)i; **C12P 19/56**(2006.01)i; **C12P 19/18**(2006.01)i; **C12N 15/62**(2006.01)i; **C12N 15/81**(2006.01)i

According to International Patent Classification (IPC) or to both national classification and IPC

**B. FIELDS SEARCHED**

Minimum documentation searched (classification system followed by classification symbols)

C12N 9/10(2006.01); C12N 15/00(2006.01); C12N 15/52(2006.01); C12N 15/70(2006.01); C12N 9/88(2006.01); C12P 19/56(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 스테비올 배당체(steviol glycoside), 스테비오사이드(stevioside), 레바우디오사이드(Rebaudioside), 우리딘 다이포스페이트-글라이코실트랜퍼라제(UDP-glycosyltransferases), 변이(mutation)

**C. DOCUMENTS CONSIDERED TO BE RELEVANT**

| Category* | Citation of document, with indication, where appropriate, of the relevant passages | Relevant to claim No. |
|---|---|---|
| A | KR 10-2021-0153670 A (CONAGEN INC.) 17 December 2021 (2021-12-17) See abstract; and claims 1-18. | 1-17 |
| A | NCBI. GenBank Accession No. XP_044372292.1. putative UDP-rhamnose:rhamnosyltransferase 1 [Triticum aestivum]. 25 October 2021. See entire document. | 1-17 |
| A | KR 10-2013-0122603 A (EVOLVA NUTRITION, INC.) 07 November 2013 (2013-11-07) See abstract; and claims 1-16. | 1-17 |
| A | US 2021-0009968 A1 (MANUS BIO, INC.) 14 January 2021 (2021-01-14) See entire document. | 1-17 |
| A | US 2018-0223264 A1 (CODEXIS, INC.) 09 August 2018 (2018-08-09) See entire document. | 1-17 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **28 March 2023** | **29 March 2023** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

**INTERNATIONAL SEARCH REPORT**

International application No.
**PCT/KR2022/021327**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

**INTERNATIONAL SEARCH REPORT**
**Information on patent family members**

International application No.
**PCT/KR2022/021327**

| Patent document cited in search report | Publication date (day/month/year) | Patent family member(s) | Publication date (day/month/year) |
|---|---|---|---|
| KR 10-2021-0153670 A | 17 December 2021 | BR 112021021828 A2 | 04 January 2022 |
| | | CN 111868252 A | 30 October 2020 |
| | | EP 3765627 A1 | 20 January 2021 |
| | | EP 3997233 A1 | 18 May 2022 |
| | | JP 2021-515555 A | 24 June 2021 |
| | | JP 2022-540306 A | 15 September 2022 |
| | | US 10883130 B2 | 05 January 2021 |
| | | US 11441165 B2 | 13 September 2022 |
| | | US 11447808 B2 | 20 September 2022 |
| | | US 2020-0002742 A1 | 02 January 2020 |
| | | US 2020-0087695 A1 | 19 March 2020 |
| | | US 2021-0189448 A1 | 24 June 2021 |
| | | WO 2019-178116 A1 | 19 September 2019 |
| | | WO 2021-007423 A1 | 14 January 2021 |
| KR 10-2013-0122603 A | 07 November 2013 | AU 2011-261394 A1 | 10 January 2013 |
| | | AU 2011-261394 B2 | 27 August 2015 |
| | | AU 2015-261617 A1 | 17 December 2015 |
| | | AU 2015-261617 B2 | 26 October 2017 |
| | | BR 112012030836 A2 | 08 October 2019 |
| | | BR 122021005283 B1 | 22 February 2022 |
| | | CA 2802627 A1 | 08 December 2011 |
| | | CA 3176307 A1 | 08 December 2011 |
| | | CL 2012003373 A1 | 08 August 2014 |
| | | CN 103179850 A | 26 June 2013 |
| | | CN 103179850 B | 16 March 2016 |
| | | CN 105671108 A | 15 June 2016 |
| | | EP 2575432 A1 | 10 April 2013 |
| | | EP 2575432 B1 | 14 August 2019 |
| | | EP 3593633 A1 | 15 January 2020 |
| | | JP 2013-533736 A | 29 August 2013 |
| | | JP 2017-074058 A | 20 April 2017 |
| | | JP 2017-079739 A | 18 May 2017 |
| | | JP 2019-146573 A | 05 September 2019 |
| | | JP 2021-151238 A | 30 September 2021 |
| | | JP 6110551 B1 | 05 April 2017 |
| | | JP 6177127 B2 | 09 August 2017 |
| | | JP 6509188 B2 | 08 May 2019 |
| | | KR 10-1971818 B1 | 23 April 2019 |
| | | KR 10-2017-0131717 A | 29 November 2017 |
| | | KR 10-2019-0043637 A | 26 April 2019 |
| | | KR 10-2181638 B1 | 24 November 2020 |
| | | MX 2012014016 A | 01 October 2013 |
| | | MX 2020007169 A | 31 August 2020 |
| | | MX 349121 B | 12 July 2017 |
| | | MY 167872 A | 26 September 2018 |
| | | NZ 604915 A | 31 October 2014 |
| | | SG 10201709458 A | 28 December 2017 |
| | | US 10392644 B2 | 27 August 2019 |
| | | US 2013-0171328 A1 | 04 July 2013 |
| | | US 2017-0218419 A1 | 03 August 2017 |

Form PCT/ISA/210 (patent family annex) (July 2022)

**INTERNATIONAL SEARCH REPORT**
**Information on patent family members**

International application No.
**PCT/KR2022/021327**

| Patent document cited in search report | Publication date (day/month/year) | Patent family member(s) | Publication date (day/month/year) |
|---|---|---|---|
| | | US 2020-0140912 A1 | 07 May 2020 |
| | | US 2022-0073960 A1 | 10 March 2022 |
| | | US 9562251 B2 | 07 February 2017 |
| | | WO 2011-153378 A1 | 08 December 2011 |
| US 2021-0009968 A1 | 14 January 2021 | AU 2020-302789 A1 | 27 January 2022 |
| | | BR 112021026306 A2 | 06 September 2022 |
| | | CA 3144658 A1 | 30 December 2020 |
| | | CN 114174501 A | 11 March 2022 |
| | | EP 3990628 A1 | 04 May 2022 |
| | | IL 289202 A | 01 February 2022 |
| | | JP 2022-530706 A | 30 June 2022 |
| | | KR 10-2022-0034793 A | 18 March 2022 |
| | | US 11168309 B2 | 09 November 2021 |
| | | US 2022-0090031 A1 | 24 March 2022 |
| | | WO 2020-264179 A1 | 30 December 2020 |
| US 2018-0223264 A1 | 09 August 2018 | AR 110954 A1 | 22 May 2019 |
| | | AU 2018-215335 A1 | 01 August 2019 |
| | | AU 2018-215335 B2 | 01 October 2020 |
| | | AU 2020-289865 A1 | 28 January 2021 |
| | | AU 2020-289868 A1 | 28 January 2021 |
| | | BR 112019015992 A2 | 26 May 2020 |
| | | CA 3050310 A1 | 09 August 2018 |
| | | CN 110914445 A | 24 March 2020 |
| | | EA 201991758 A1 | 25 March 2020 |
| | | EP 3577229 A2 | 11 December 2019 |
| | | EP 3577229 A4 | 23 December 2020 |
| | | EP 3878967 A2 | 15 September 2021 |
| | | EP 3878967 A3 | 08 December 2021 |
| | | EP 3882354 A2 | 22 September 2021 |
| | | EP 3882354 A3 | 15 December 2021 |
| | | IL 268121 A | 26 September 2019 |
| | | JP 2020-506704 A | 05 March 2020 |
| | | JP 2021-036911 A | 11 March 2021 |
| | | JP 2021-087447 A | 10 June 2021 |
| | | KR 10-2019-0124723 A | 05 November 2019 |
| | | MX 2019009233 A | 19 September 2019 |
| | | PH 12019501698 A1 | 04 November 2019 |
| | | SG 11201906480 A | 27 August 2019 |
| | | TW 201837171 A | 16 October 2018 |
| | | US 2021-0054348 A1 | 25 February 2021 |
| | | US 2021-0054349 A1 | 25 February 2021 |
| | | WO 2018-144675 A1 | 09 August 2018 |
| | | WO 2018-144679 A2 | 09 August 2018 |
| | | WO 2018-144679 A3 | 13 December 2018 |

Form PCT/ISA/210 (patent family annex) (July 2022)